# EUROPÄISCHE PATENTSCHRIFT

(11) **EP 1 054 009 B1**
(45) Veröffentlichungstag und Bekanntmachung des Hinweises auf die Patenterteilung: **27.11.2002**
(21) Anmeldenummer: 00108912.7
(22) Anmeldetag: 27.04.2000
(51) Int. Cl.: C07D 311/76, C07C 67/307

(54) **Verfahren zur Herstellung von 3-Isochromanonen durch Cyclisierung von o-Chlormehtylphenylessigsäuren**
Process for preparation of 3-isochromanones by cyclisation of o-chloromethylphenylacetic acids
Procédé de préparation de 3-isochromanones par cyclisation d' acides o-chlorométhylphénylacétiques

(30) Priorität: 21.05.1999 DE 19923548
(43) Veröffentlichungstag der Anmeldung: 22.11.2000
(73) Patentinhaber: Clariant GmbH, 65929 Frankfurt am Main (DE)
(72) Erfinder: Geissler, Holger, Dr., 55116 Mainz (DE)

(56) Entgegenhaltungen:
- WO-A-97/48692
- "SOME INTERMEDIATES AND ROUTES FOR THE PREPARATION OF 3-ISOCHROMANONE" RESEARCH DISCLOSURE., Bd. 409, Mai 1998 (1998-05), Seiten 581-4, XP000824703 INDUSTRIAL OPPORTUNITIES LTD. HAVANT., GB ISSN: 0374-4353

## Beschreibung

Verfahren zur Herstellung von 3-lsochromanonen durch Cyclisierung von o-Chlormethyl-phenylessigsäuren

Die vorliegende Erfindung betrifft ein neues, vorteilhaftes Verfahren zur Herstellung von 3-Isochromanonen durch Cyclisierung von o-Chlormethyl-phenylessigsäuren. Isochroman-3-on ist bei der Synthese von Pharmazeutika und Pflanzenschutzmitteln als Zwischenprodukt von großem Interesse.

Aus der WO 97/12864 geht beispielsweise die Verwendung von 3-Isochromanon als Zwischenprodukt bei der Herstellung von Fungiziden und Pestiziden und aus WO 97/48692 die Verwendung von 3-Isochromanon bei Herstellung bestimmter landwirtschaftlicher Produkte hervor.

3-Isochromanon ist eine bekannte Verbindung und eine Vielzahl von Methoden zur Darstellung ist in WO 97/48692 erwähnt. Zum Beispiel kann 3-Isochromanon durch Baeyer-Villiger-Oxidation von 2-Indanon unter Verwendung von Wasserstoffperoxid in Schwefelsäure und Essigsäureanhydrid oder unter Verwendung von m-Chlorperbenzoesäure in Kombination mit Trifluoressigsäure oder durch Umsetzung von Brommethyl-phenylessigsäure mit KOH in Ethanol unter Ringschluß hergestellt werden.

WO 97/48692 offenbart eine Synthese von 3-Isochromanon durch Chlorierung von o-Methyl-phenylessigsäure mit Sulfurylchlorid in Gegenwart von Radikalinitiatoren und durch anschließende Umsetzung der entstandenen 2-Chlormethyl-phenylessigsäure mit einer Base. Der Nachteil dieses Verfahrens ist die Bildung von einem Äquivalent Salz pro Äquivalent 3-lsochromanon bei der Umsetzung von 2-Chlormethyl-phenylessigsäure unter Einsatz einer Base und die Handhabung Salze enthaltender Reaktionsgemische.

Daher besteht ein Bedarf nach einem Verfahren, das die genannten Nachteile nicht aufweist, für eine technische Durchführung geeignet ist und 3-Isochromanon in guter Ausbeute liefert.

Diese Aufgabe wird gelöst durch ein Verfahren zur Herstellung eines 3-lsochromanons der allgemeinen Formel (I) 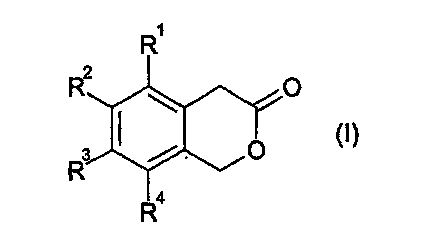 durch Umsetzung einer o-Chlormethyl-phenylessigsäure der allgemeinen Formel (II) 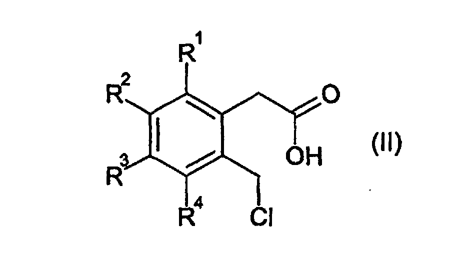 bei einer Temperatur von 100 bis 250 °C in Anwesenheit oder Abwesenheit eines ionischen Halogenids, in Anwesenheit oder Abwesenheit eines organischen Lösemittels und Abtrennung von Chlorwasserstoff, wobei in den Formeln (I) und (II) die Reste R¹, R², R³ und R⁴ unabhängig voneinander darstellen:
ein Wasserstoff- oder Fluoratom;
eine NC- oder F₃C-Gruppe;
einen Alkyl-, Alkoxy- oder Acyloxyrest, mit jeweils 1 bis 18 Kohlenstoffatomen; oder
einen C₆-C₁₈-Aryloxy-, Aryl- oder Heteroarylrest, wobei als Heteroatome 1 bis 3 Atome aus der Gruppe O, N und/oder S vorhanden sind;
oder worin mindestens zwei der Reste R¹, R², R³ und R⁴ untereinander verknüpft sind und mindestens einen aliphatischen oder aromatischen Ring mit 5 bis 18 C-Atomen bilden.

Überraschenderweise und entgegen den Anweisungen aus der Literatur (WO 97/48692), die Ringschlußreaktion von Brom/Chlormethyl-phenylessigsäure durch Zugabe einer Base herbeizuführen, erhält man nach dem erfindungsgemäßen Verfahren 3-Isochromanone durch Umsetzung einer o-Chlormethyl-phenylessigsäure der Formel (II) unter Ringschluß in Abwesenheit einer Base. Hierbei wird die o-Chlormethyl-phenylessigsäure nicht durch Umsetzung mit einer Base in das entsprechende Salz der o-Chlormethyl-phenylessigsäure überführt und aus dem Salz durch Cyclisierung das entsprechende 3-Isochromanon gebildet, sondern wird vermutlich der Chlorwasserstoff direkt aus der o-Chlormethyl-phenylessigsäure unter gleichzeitigem Ringschluß und Bildung des entsprechenden 3-lsochromanons abgespalten.
Die Umsetzung verläuft somit nicht über ein Salz der o-Chtormethylphenylessigsäure als Zwischenprodukt, das anschließend cyclisiert wird, sondern führt unter Abspaltung von Chlorwasserstoff zum entsprechenden 3-Isochromanon.
Der Chlorwasserstoff wird üblicherweise entsprechend seiner Bildung aus dem Reaktionsgemisch auf thermischen Wege entfernt. Man kann aus dem Reaktionsgemisch, das ein organisches Lösemittel enthalten kann, den Chlorwasserstoff oder ein den Chlorwasserstoff und das organische Lösemittel enthaltenes Gemisch abdampfen. Trennt man ein derartiges Gemisch ab, so empfiehlt es sich, das organische Lösemittel zu kondensieren, den Chlorwasserstoff abzutrennen und, falls gewünscht, das vom Chlorwasserstoff befreite Lösemittel wieder in die Reaktion zurückzuführen.

Die Reste R¹, R², R³ und R⁴ bedeuten unabhängig voneinander insbesondere Wasserstoff, Fluor, C₁-C₄-Alkyl oder C₁-C₄-Alkoxy, bevorzugt Wasserstoff, Fluor, C₁-C₄-n-Alkyl oder C₁-C₄-n-Alkoxy, oder zwei der Reste R¹, R², R³ und R⁴ sind untereinander verknüpft und bilden einen aliphatischen oder aromatischen Ring mit 5 bis 10 C-Atomen. Vorzugsweise stehen R¹, R², R³ und R⁴ unabhängig voneinander für Wasserstoff, Fluor oder C₁-C₄-Alkyl, insbesondere für Wasserstoff, Fluor oder C₁-C₄-n-Alkyl.

In den Formeln (I) und (II) bedeuten einer besonderen Ausführungsform zufolge zwei, drei oder vier, insbesondere drei oder vier der Reste R¹, R², R³ und R⁴ Wasserstoff.

Wie eingangs erwähnt, kann man das Verfahren in Anwesenheit oder Abwesenheit eines ionischen Halogenids durchführen.

Üblicherweise ist das ionische Halogenid ein Alkali-, Ammonium- oder Phosphoniumhalogenid, insbesondere ein Alkali- oder Ammoniumhalogenid, wobei Halogenid die Bedeutung Chlorid, Bromid oder lodid, insbesondere lodid oder Bromid hat.

Man kann als ionisches Halogenid Ammoniumbromid, Lithiumbromid, Natriumbromid, Kaliumbromid, Tetrabutylphosphoniumbromid, Ammoniumchlorid, Dimethylammoniumchlorid, Diethanolammoniumchlorid, Lithiumchlorid, Natriumchlorid, Kaliumchlorid, Tetrabutylphosphoniumchlorid, Ammoniumjodid, Lithiumjodid, Natriumjodid, Kaliumjodid und/oder Tetrabutylphosphoniumjodid einsetzen.

Das ionische Halogenid wird in der Regel in einer Menge von 0,005 bis 0,5 Äquivalenten, insbesondere von 0,01 bis 0,05 Äquivalenten pro Äquivalent eingesetzter o-Chlormethyl-phenylessigsäure eingesetzt.

An dieser Stelle sei darauf hingewiesen, daß man auf die Anwesenheit des ionischen Halogenids verzichten und das Verfahren insbesondere in Abwesenheit des ionischen Halogenids durchführen kann.

In der Regel erfolgt die Umsetzung bei einem Druck von 10 Pa bis 0,5 MPa, insbesondere bei 100 Pa bis 0,2 MPa, bevorzugt bei 1000 Pa bis 0,12 MPa.

Man führt die Umsetzung - wie eingangs bereits erwähnt - bei einer Temperatur von 100 bis 250°C, insbesondere 120 bis 220°C, bevorzugt 150 bis 200°C durch. Druck und Temperatur werden so gewählt, daß der bei der Umsetzung entstehende Chlorwasserstoff gasförmig anfällt und in gasförmigem Zustand aus dem Reaktionsgemisch abgetrennt werden kann.

Wie eingangs erwähnt, kann das Verfahren in Anwesenheit oder Abwesenheit eines organischen Lösemittels durchgeführt werden.

Als organisches Lösemittel eignen sich insbesondere dipolar aprotische Lösemittel wie Dioxan, Tetrahydrofuran, ein N-(C₁-C₁₈-Alkyl)pyrrolidon, Ethylenglykoldimethylether, ein C₁-C₄-Alkylester einer aliphatischen C₁-C₆-Carbonsäure, ein C₁-C₆-Dialkylether, ein N,N-Di-(C₁-C₄-alkyl)amid einer aliphatischen C₁-C₄-Carbonsäure, Sulfolan, ein 1,3-Di-(C₁-C₈-alkyl)-2-imidazolidinon, ein N-(C₁-C₈-Alkyl)caprolactam, ein N,N,N',N'-Tetra-(C₁-C₈-alkyl)hamstoff, ein 1,3-Di-(C₁-C₈-alkyl)-3,4,5,6-tetrahydro-2(1H)-pyrimidon, ein N,N,N',N'-Tetra-(C₁-C₈alkyl)sulfamid, 4-Formylmorpholin, 1-Formylpiperidin oder 1-Formylpyrrolidin, bevorzugt ein N-(C₁-C₁₈-Alkyl)pyrrolidon, besonders bevorzugt N-Methylpyrrolidon, N-Octylpyrrolidon, N-Dodecylpyrrolidon, ganz besonders bevorzugt N-Methylpyrrolidon. Es lassen sich auch Mischungen von Lösemitteln verwenden.

In einer bevorzugten Ausführungsform des erfindungsgemäßen Verfahrens werden Druck und Temperatur so gewählt, daß das eingesetzte Lösemittel siedet. Das verdampfte Lösemittel wird kondensiert, vom Chlorwasserstoff abgetrennt und üblicherweise in die Reaktion zurückgeführt.

Der sich in der Reaktion bildende Chlorwasserstoff entweicht in der Regel gasförmig aus der Reaktionsmischung und kann in herkömmlichen Verfahren mittels Absorption zurückgewonnen und wiedereingesetzt werden. So erhält man durch Absorption von gasförmigen Chlorwasserstoff in Wasser eine wässrige Salzsäure, die in anderen technischen Prozessen einsetzbar ist Bei der Verwendung von organischen Lösemitteln gelingt das Abtrennen des gasförmigen Chlorwasserstoffs besonders einfach am Siedepunkt der eingesetzten organischen Lösemittel. So läßt sich die Reaktion z. B. in siedendem N-Methylpyrrolidon bei 160 °C und 0,025 MPa besonders gut durchführen.

Die Dauer der Reaktion hängt unter anderem von der Reaktionstemperatur und bei der Verwendung eines organischen Lösemittels von der Löslichkeit des Chlorwasserstoffes in dem eingesetzten Lösemittel bei der entsprechenden Reaktionstemperatur ab.

Während der Umsetzung sorgt man für eine gute Durchmischung, um einen zügigen Reaktionsverlauf zu gewährleisten.

Das erfindungsgemäße Verfahren eignet sich sowohl für eine kontinuierliche als auch diskontinuierliche Durchführung.

Der nachfolgende experimentelle Teil beschreibt die Erfindung näher, ohne sie zu beschränken.

### Experimenteller Teil

### Beispiel 1

Die Reaktionsapparatur besteht aus einem Reaktionsgefäß (1 Liter Glasgefäß), bestückt mit Rührer, einer mit Raschigringen befüllten Kolonne (25 cm), einem oberhalb der Kolonne angeordneten Rückflußkühler (Kondensiervorrichtung) und einer über eine Leitung mit dem Rückflußkühler verbundenen Waschkolonne, in die von unten gasförmiger Chlorwasserstoff eingeleitet wird, während von oben im Gegenstrom Wasser durch die Waschkolonne geleitet wird, um Chlorwasserstoff unter Bildung wässriger Salzsäure zu absorbieren. Die wässrige Salzsäure wird am Boden der Waschkolonne abgezogen. Der Kopf der Waschkolonne ist mit einer Vakuumpumpe verbunden.

### Durchführung der Reaktion:

46,0 g o-Chlormethyl-phenylessigsäure werden im Reaktionsgefäß in 415 g N-Methylpyrrolidon gelöst und bei einer Temperatur von 160°C und 0,025 MPa 6 Stunden lang gerührt. Die Temperatur in dem Rückflußkühler wird auf 80°C eingestellt und der Druck mittels der Vakuumpumpe konstant bei 0,025 MPa gehalten. Gasförmiger Chlorwasserstoff gelangt zusammen mit verdampftem Lösemittel über die mit Raschigringen befüllte Kolonne in den Rückflußkühler, wo kondensierbare Anteile kondensieren und in das Reaktionsgefäß zurückfließen, während gasförmiger Chlorwasserstoff der nachgeschalteten Waschkolonne zugeführt wird. Eine HPLC-Analyse des Reaktionsgemisches zeigt, daß die Reaktionsmischung 31,9 g 3-lsochromanon (Ausbeute 86,5%) enthält. Zur Aufarbeitung wird nach Abschluß der Reaktion das Lösemittel N-Methylpyrrolidon bei 100 °C und 20 hPa von der Reaktionsmischung abgedampft und anschließend wird bei 160 °C und 5 hPa (Kopftemperatur 144°C) das entstandene 3-Isochromanon aus dem Sumpf destilliert. Man erhält 30,3 g 3-Isochromanon (Ausbeute 82%).

## Patentansprüche

1. Verfahren zur Herstellung eines 3-Isochromanons der allgemeinen Formel (I) 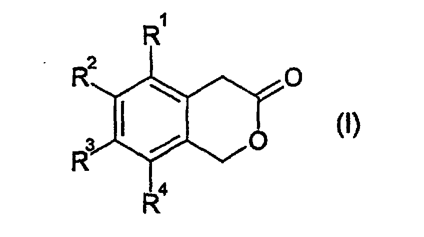 durch Umsetzung einer o-Chlormethyl-phenylessigsäure der allgemeinen Formel (II) 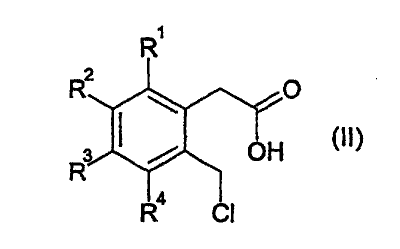 in Abwesenheit einer Base bei einer Temperatur von 100 bis 250 °C in Anwesenheit oder Abwesenheit eines ionischen Halogenids, in Anwesenheit oder Abwesenheit eines organischen Lösemittels und Abtrennung von Chlorwasserstoff, wobei in den Formeln (I) und (II) die Reste R¹, R², R³ und R⁴ unabhängig voneinander darstellen:
ein Wasserstoff- oder Fluoratom;
eine NC- oder F₃C-Gruppe;
einen Alkyl-, Alkoxy- oder Acyloxyrest, mit jeweils 1 bis 18 Kohlenstoffatomen; oder
einen C₆-C₁₈-Aryloxy-, Aryl- oder Heteroarylrest, wobei als Heteroatome 1 bis 3 Atome aus der Gruppe O, N und/oder S vorhanden sind;
oder worin mindestens zwei der Reste R¹, R², R³ und R⁴ untereinander verknüpft sind und mindestens einen aliphatischen oder aromatischen Ring mit 5 bis 18 C-Atomen bilden.

2. Verfahren nach Anspruch 1, **dadurch gekennzeichnet, daß** die Reste R¹, R², R³ und R⁴ unabhängig voneinander Wasserstoff, Fluor, C₁-C₄-Alkyl oder C₁-C₄-Alkoxy bedeuten oder zwei der Reste R¹, R², R³ und R⁴ untereinander verknüpft sind und einen aliphatischen oder aromatischen Ring mit 5 bis 10 C-Atomen bilden.

3. Verfahren nach Anspruch 1 oder 2, **dadurch gekennzeichnet, dass** zwei, drei oder vier der Reste R¹, R², R³ und R⁴ Wasserstoff bedeuten.

4. Verfahren nach einem oder mehreren der Ansprüche 1 bis 3, **dadurch gekennzeichnet, dass** das ionische Halogenid ein Alkali-, Ammonium- oder Phosphoniumhalogenid ist, wobei Halogenid die Bedeutung Chlorid, Bromid oder lodid hat.

5. Verfahren nach einem oder mehreren der Ansprüche 1 bis 4, **dadurch gekennzeichnet, dass** das ionische Halogenid Ammoniumbromid, Lithiumbromid, Natriumbromid, Kaliumbromid, Tetrabutylphosphoniumbromid, Ammoniumchlorid, Dimethylammoniumchlorid, Diethanolammoniumchlorid, Lithiumchlorid, Natriumchlorid, Kaliumchlorid, Tetrabutylphosphoniumchlorid, Ammoniumiodid, Lithiumiodid, Natriumiodid, Kaliumiodid und/oder Tetrabutylphosphoniumiodid ist.

6. Verfahren nach einem oder mehreren der Ansprüche 1 bis 5, **dadurch gekennzeichnet, dass** man ein dipolar aprotisches Lösemittel als organisches Lösemittel einsetzt.

7. Verfahren nach einem oder mehreren der Ansprüche 1 bis 6, **dadurch gekennzeichnet, dass** das dipolar aprotische Lösemittel Dioxan, Tetrahydrofuran, ein N-(C₁-C₁₈-Alkyl)pyrrolidon, Ethylenglykoldimethylether, ein C₁-C₄-Alkylester einer aliphatischen C₁-C₆-Carbonsäure, ein C₁-C₆-Dialkylether, ein N,N-Di-(C₁-C₄alkyl)amid einer aliphatischen C₁-C₄-Carbonsäure, Sulfolan, 1,3-Di-(C₁-C₈-alkyl)-2-imidazolidinon, ein N-(C₁-C₈-Alkyl)caprolactam, ein N,N,N',N'-Tetra-(C₁-C₈alkyl)harnstoff, ein 1,3-Di-(C₁-C₈-alkyl)-3,4,5,6-tetrahydro-2(1H)-pyrimidon, ein N,N,N',N'-Tetra-(C₁-C₈-alkyl)sulfamid, 4-Formylmorpholin, 1-Formylpiperidin oder 1-Formylpyrrolidin ist.

8. Verfahren nach einem oder mehreren der Ansprüche 1 bis 7, **dadurch gekennzeichnet, dass** das dipolar aprotische Lösemittel ein N-(C₁-C₁₈-Alkyl)pyrrolidon ist.

9. Verfahren nach einem oder mehreren der Ansprüche 1 bis 8, **dadurch gekennzeichnet, dass** das dipolar aprotische Lösemittel N-Methylpyrrolidon, N-Octylpyrrolidon oder N-Dodecylpyrrolidon ist.

## Claims

1. A process for the preparation of a 3-isochromanone of the formula (I) 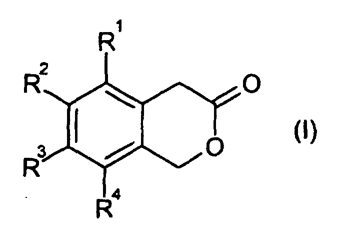 by reaction of an o-chloromethylphenylacetic acid of the formula (II) 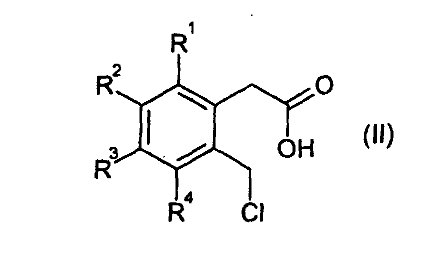 in the absence of a base and at a temperature of 100 to 250°C in the presence or absence of an ionic halide, in the presence or absence of an organic solvent and with removal of hydrogen chloride, where in the formulae (I) and (II) the radicals R¹, R², R³ and R⁴ independently of one another are:
a hydrogen or fluorine atom;
an NC or F₃C group;
an alkyl, alkoxy or acyloxy radical, each having 1 to 18 carbon atoms; or a C₆-C₁₈-aryloxy, aryl or heteroaryl radical, 1 to 3 atoms from the group consisting of O, N and/or S being present as heteroatoms;
or in which at least two of the radicals R¹, R², R³ and R⁴ are linked to one another and form at least one aliphatic or aromatic ring having 5 to 18 carbon atoms.

2. The process as claimed in claim 1, wherein the radicals R¹, R², R³ and R⁴ independently of one another are hydrogen, fluorine, C₁-C₄-alkyl or C₁-C₄-alkoxy or two of the radicals R¹, R², R³ and R⁴ are linked to one another and form an aliphatic or aromatic ring having 5 to 10 carbon atoms.

3. The process as claimed in claim 1 or 2, wherein two, three or four of the radicals R¹, R², R³ and R⁴ are hydrogen.

4. The process as claimed in one or more of claims 1 to 3, wherein the ionic halide is an alkali metal, ammonium or phosphonium halide, halide having the meaning chloride, bromide or iodide.

5. The process as claimed in one or more of claims 1 to 4, wherein the ionic halide is ammonium bromide, lithium bromide, sodium bromide, potassium bromide, tetrabutylphosphonium bromide, ammonium chloride, dimethylammonium chloride, diethanolammonium chloride, lithium chloride, sodium chloride, potassium chloride, tetrabutylphosphonium chloride, ammonium iodide, lithium iodide, sodium iodide, potassium iodide and/or tetrabutylphosphonium iodide.

6. The process as claimed in one or more of claims 1 to 5, wherein a dipolar aprotic solvent is employed as an organic solvent.

7. The process as claimed in one or more of claims 1 to 6, wherein the dipolar aprotic solvent is dioxane, tetrahydrofuran, an N-(C₁-C₁₈-alkyl)pyrrolidone, ethylene glycol dimethyl ether, a C₁-C₄-alkyl ester of an aliphatic C₁-C₆-carboxylic acid, a C₁-C₆-dialkyl ether, an N,N-di(C₁-C₄-alkyl)amide of an aliphatic C₁-C₄-carboxylic acid, sulfolane, a 1,3-di(C₁-C₈-alkyl)-2-imidazolidinone, an N-(C₁-C₈-alkyl)caprolactam, an N,N,N',N'-tetra(C₁-C₈-alkyl)-urea, a 1,3-di-(C₁-C₈-alkyl)-3,4,5,6-tetrahydro-2(1H)-pyrimidone, an N,N,N',N'-tetra-(C₁-C₈-alkyl)-sulfamide, 4-formylmorpholine, 1-formylpiperidine or 1-formylpyrrolidine.

8. The process as claimed in one or more of claims 1 to 7, wherein the dipolar aprotic solvent is an N-(C₁-C₁₈-alkyl)pyrrolidone.

9. The process as claimed in one or more of claims 1 to 8, wherein the dipolar aprotic solvent is N-methylpyrrolidone, N-octylpyrrolidone or N-dodecylpyrrolidone.

## Revendications

1. Procédé pour la préparation d'une 3-isochromanone de formule générale (I) 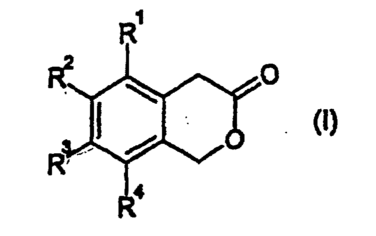 par réaction d'un acide o-chlorométhyl-phénylacétique de formule générale (II) 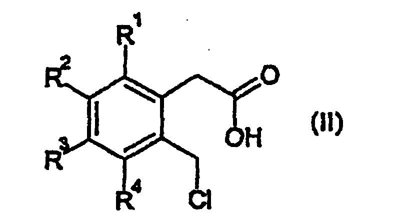 en l'absence d'une base, à une température de 100 à 250°C, en présence ou en l'absence d'un halogénure ionique, en présence ou en l'absence d'un solvant organique et par séparation du chlorure d'hydrogène, où les restes R¹, R², R³ et R⁴ dans les formules (I) et (II) représentent, indépendamment l'un de l'autre :
un atome d'hydrogène ou un atome de fluor ;
un groupe NC ou F₃C ;
un reste alkyle, alkoxy ou acyloxy, chacun présentant 1 à 18 atomes de carbone ; ou un reste hétéroaryle, aryle ou (aryl en C₆-C₁₈)-oxy, 1 à 3 atomes pris dans le groupe comprenant des atomes de O, N et/ou S étant présents en tant qu'hétéroatomes ;
ou au moins deux des restes R¹, R², R³ et R⁴ sont reliés entre eux et forment au moins un cycle aliphatique ou aromatique présentant 5 à 18 atomes de carbone.

2. Procédé selon la revendication 1, **caractérisé en ce que** les restes R¹, R², R³ et R⁴ représentent, indépendamment les uns des autres, des atomes d'hydrogène, des atomes de fluor, des groupes alkyle en C₁-C₄ ou alkoxy en C₁-C₄ ou deux des restes R¹, R², R³ et R⁴ sont reliés entre eux et forment un cycle aliphatique ou aromatique ayant 5 à 10 atomes de carbone.

3. Procédé selon la revendication 1 ou 2, **caractérisé en ce que** deux, trois ou quatre des restes R¹, R², R³ et R⁴ représentent des atomes d'hydrogène.

4. Procédé selon une ou plusieurs des revendications 1 à 3, **caractérisé en ce que** l'halogénure ionique est un halogénure alcalin, un halogénure d'ammonium ou de phosphonium, l'halogénure étant un chlorure, un bromure ou un iodure.

5. Procédé selon une ou plusieurs des revendications 1 à 4, **caractérisé en ce que** l'halogénure ionique est le bromure d'ammonium, le bromure de lithium, le bromure de sodium, le bromure de potassium, le bromure de tétrabutylphosphonium, le chlorure d'ammonium, le chlorure de diméthylammonium, le chlorure de diéthanolammonium, le chlorure de lithium, le chlorure de sodium, le chlorure de potassium, le chlorure de tétrabutylphosphonium, l'iodure d'ammonium, l'iodure de lithium, l'iodure de sodium, l'iodure de potassium et/ou l'iodure de tétrabutylphosphonium.

6. Procédé selon une ou plusieurs des revendications 1 à 5, **caractérisé en ce qu'**on utilise un solvant aprotique dipolaire en tant que solvant organique.

7. Procédé selon une ou plusieurs des revendications 1 à 6, **caractérisé en ce que** le solvant aprotique dipolaire est le dioxanne, le tétrahydrofuranne, une N-(alkyl en C₁-C₁₈)pyrrolidone, l'éther diméthylique de l'éthylèneglycol, un ester d'alkyle en C₁-C₄ d'un acide carboxylique en C₁-C₆ aliphatique, un éther dialkylique en C₁-C₆, un N,N-di-(alkyl en C₁-C₄)-amide d'un acide carboxylique en C₁-C₄ aliphatique, la sulfolane, la 1,3-di-(alkyl en C₁-C₈)-2-imidazolinone, un N-(alkyl en C₁-C₈)-caprolactame, une N,N,N',N'-tétra-(alkyl en C₁-C₈)-urée, une la 1,3-di-(alkyl en C₁-C₈)-3,4,5,6-tétrahydro-2(1H)-pyrimidone, un N,N,N',N'-tétra-(alkyl en C₁-C₈)-sulfamide, la 4-formylmorpholine, la 1-formylpipéridine ou 1-formylpyrrolidine.

8. Procédé selon une ou plusieurs des revendications 1 à 7, **caractérisé en ce que** le solvant aprotique dipolaire est une N-(alkyl en C₁-C₁₈)-pyrrolidone.

9. Procédé selon une ou plusieurs des revendications 1 à 8, **caractérisé en ce que** le solvant aprotique dipolaire est la N-méthylpyrrolidone, la N-octyipyrrolidone ou la N-dodécylpyrrolidone.
